# EUROPEAN PATENT APPLICATION

(11) **EP 4 672 153 A1**
(43) Date of publication of application: **31.12.2025**
(21) Application number: 25184839.6
(22) Date of filing: 24.06.2025
(51) Int. Cl.: G06T 11/00, A61B 6/00

(54) **POSITIONING SCAN METHOD AND APPARATUS, DUAL-SOURCE CT APPARATUS, MEDIUM AND PRODUCT**

(30) Priority: 27.06.2024 CN 202410854123
(71) Applicant: Siemens Healthineers AG, 91301 Forchheim (DE)
(72) Inventor: SHANG, Lei Min, Shanghai, 200120 (CN); WANG, Jing Bo, Shanghai, 200010 (CN)
(74) Representative: HKW Intellectual Property PartG mbB

(57) **Abstract**

Embodiments of the present invention disclose a positioning scan method and apparatus, a dual-source CT apparatus, a medium and a product. A first tube and a second tube in the dual-source CT apparatus have automatically returned to initial positions before a positioning scan is performed, wherein initial positions of the first tube and the second tube are respectively located directly above/below a gantry, and at a left/right lateral position on the gantry; the positioning scan method comprises: receiving a positioning scan instruction; performing the following operations according to a type of the positioning scan instruction: if it is a front image scan instruction, controlling the first tube to perform exposure, to obtain first front projection data; if it is a lateral image scan instruction, controlling the second tube to perform exposure, to obtain first lateral projection data; if it is a dual positioning image scan instruction, controlling the first tube and the second tube to simultaneously perform exposure, to obtain second front projection data and second lateral projection data respectively. Embodiments of the present invention are intended to solve the problem of low working efficiency of existing positioning scan methods for dual-source CT apparatuses.

## Description

### TECHNICAL FIELD

The present invention relates to the technical field of medical equipment, in particular to a positioning scan method and apparatus, a dual-source CT apparatus, a non-transitory computer-readable storage medium and a computer program product.

### BACKGROUND ART

CT (Computed Tomography) technology is an examination technique that is commonly used in many fields, such as medicine. A CT apparatus is a complex diagnostic system consisting of many high-precision electrical components, mainly a tube for emitting rays, a detector, a calibrator and a high-voltage generator. A common CT apparatus is typically equipped with a ray tube system and a detector system (i.e. single-source CT), and thereby acquires an image of a human body. To increase temporal resolution and further improve the quality of CT images, dual-source CT (dual-source computed tomography, DSCT) has also been proposed at the present time. Dual-source CT is a CT apparatus that uses two ray tube systems and two detector systems to acquire images of the human body simultaneously.

To perform scanning and imaging of the human body using the CT apparatus, it is necessary to place the examination subject on a bed board of a patient examination table, and move the bed board to deliver the examination subject in a horizontal state into the scanning region of the CT gantry, and only then can the scanning examination be performed. The scanning examination generally includes two scanning stages: the first stage is a positioning scan (topo scan), i.e. a stage of determining the scanning range, in which the start position, scan length and dosage scheme of a tomography scan (tomo scan) are determined according to the acquired positioning scan image. The second stage is the tomography scan, i.e. the stage of actual scanning and imaging. In the CT apparatus, the positioning scan image is also called the topo scan image.

When performing the positioning scan in the first stage, the positioning scan image required to determine the scanning range of the target organ might be different for different pre-scanned target organs or application scenarios. For example, sometimes, it might only be necessary to acquire a front positioning scan image or a lateral positioning scan image; at other times, it might be necessary to acquire a front positioning scan image and a lateral positioning scan image simultaneously (i.e. dual positioning scan images).

However, existing positioning scan methods for dual-source CT apparatuses are afflicted by the problem of low working efficiency.

### SUMMARY OF THE INVENTION

An object of the present invention is to provide a positioning scan method and apparatus, a dual-source CT apparatus, a non-transitory computer-readable storage medium and a computer program product, for the purpose of increasing the scanning efficiency of a dual-source CT apparatus in a positioning scan stage.

To achieve the above object, a first aspect of the present invention provides a positioning scan method, applied to a dual-source CT apparatus, wherein a first tube and a second tube in the dual-source CT apparatus have automatically returned to initial positions before a positioning scan is performed, and wherein an initial position of the first tube is located directly above/below a gantry, and an initial position of the second tube is located at a left/right lateral position on the gantry; the positioning scan method comprises: receiving a positioning scan instruction;
performing the following operations according to a type of the positioning scan instruction: if it is a front image scan instruction, controlling the first tube to perform exposure, to obtain first front projection data; if it is a lateral image scan instruction, controlling the second tube to perform exposure, to obtain first lateral projection data; if it is a dual positioning image scan instruction, controlling the first tube and the second tube to simultaneously perform exposure, to obtain second front projection data and second lateral projection data respectively.

This technical solution optimizes positioning scan logic in the dual-source CT apparatus by incorporating the second tube in the topo scan, reducing the duration of a tube movement process, and thereby reducing the waiting time of the positioning scan as a whole, and increasing the overall scanning efficiency.

Preferably, the positioning scan method further comprises: subjecting the second front projection data and the second lateral projection data to interference correction, to obtain the second front projection data and the second lateral projection data in corrected form; obtaining a second front positioning scan image by reconstruction on the basis of the second front projection data in corrected form, and obtaining a second lateral positioning scan image by reconstruction on the basis of the second lateral projection data in corrected form. This technical solution can eliminate projection data errors caused by overlapping of rays.

Preferably, the positioning scan method further comprises: subjecting the second lateral positioning scan image to field-of-view expansion correction, to enable it to be matched with the second front positioning scan image. This technical solution can avoid a situation where the second lateral positioning scan image has incomplete field-of-view coverage and is thus unable to be matched with the second front positioning scan image.

Preferably, the positioning scan method further comprises: obtaining a first front positioning scan image by reconstruction according to the first front projection data. This technical solution can determine a start position, a scan length and a dosage scheme of a subsequent Tomo scan according to the first front positioning scan image.

Preferably, the positioning scan method further comprises: obtaining a first lateral positioning scan image by reconstruction according to the first lateral projection data. This technical solution can determine a start position, a scan length and a dosage scheme of a subsequent Tomo scan according to the first lateral positioning scan image.

Preferably, when performing a dual positioning image scan, a first dose is used during exposure by the first tube, a second dose is used during exposure by the second tube, and the second dose is less than the first dose. This technical solution can reduce harm to the examination subject.

A second aspect of the present invention provides a dual-source CT apparatus, comprising: a first tube and a second tube, which have automatically returned to initial positions before a positioning scan is performed, wherein an initial position of the first tube is located directly above/below a gantry, and an initial position of the second tube is located at a left/right lateral position on the gantry; and a controller, configured to perform the method described in the present invention.

A third aspect of the present invention provides a non-transitory computer-readable storage medium storing a computer program, wherein the computer program, when executed by a processor, realizes the method described in the present invention.

A fourth aspect of the present invention provides a computer program product, comprising a computer program which, when executed by a processor, realizes the method described in the present invention.

In summary, compared with the prior art, the positioning scan method and apparatus, the dual-source CT apparatus, the non-transitory computer-readable storage medium and the computer program product provided in the present invention have the following beneficial effects:
When a front image scan instruction is received, exposure is performed by the first tube located directly above/below the gantry; when a lateral image scan instruction is received, exposure is performed by the second tube located at the left/right lateral position on the gantry; when a dual positioning image scan instruction is received, exposure is performed simultaneously by the first tube located directly above/below the gantry and the second tube located at the left/right lateral position on the gantry. Compared with the prior art, when performing a lateral image scan and a dual positioning image scan, there is no need for the first tube to move to a corresponding position, so the waiting time of the scan as a whole is reduced, thus increasing the overall positioning scan efficiency. Especially in the case of a dual positioning image scan, it is possible to obtain front projection data and lateral projection data simultaneously in a single exposure, significantly reducing the duration of scanning, and thus increasing the competitiveness of the dual-source CT apparatus in high-throughput clinical scenarios.

### BRIEF DESCRIPTION OF THE DRAWINGS

Preferred embodiments of the present invention will be described in detail below with reference to the drawings, to give an ordinary person skilled in the art a clearer understanding of the abovementioned and other features and advantages of the present invention. In the drawings:
Figs. 1A and 1B are schematic drawings of front image scanning and lateral image scanning, respectively, by an existing positioning scan method.
Fig. 2 is a flow chart of a positioning scan method provided in an embodiment of the present invention.
Figs. 3A and 3B are schematic drawings of lateral image scanning and dual positioning image scanning, respectively, by the positioning scan method in an embodiment of the present invention.
Fig. 4 is a structural block diagram of a positioning scan apparatus provided in an embodiment of the present invention.
Fig. 5 is a structural block diagram of a dual-source CT apparatus provided in an embodiment of the present invention.

In the figures, the reference numerals are as follows:

### Reference numeralMeaning

- 10: Gantry
- 20: Examination subject
- A, B: Tubes
- a, b: Detectors
- 100: Positioning scan method
- S110, S120: Steps
- 200: Positioning scan apparatus
- 210: Instruction receiving module
- 220: Instruction executing module
- 300: Dual-source CT apparatus
- 310: First tube
- 320: Second tube
- 330: Controller

### DETAILED DESCRIPTION OF EMBODIMENTS

To clarify the objectives, technical solutions and advantages of the present invention, the present invention will be explained in further detail below through embodiments.

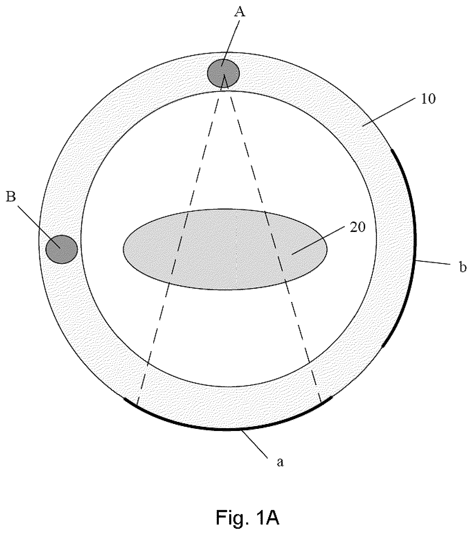

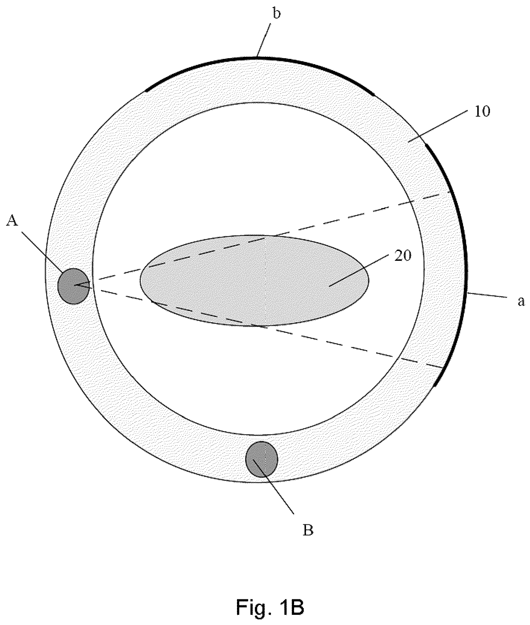

Existing positioning scan methods for dual-source CT apparatuses use only one of the tubes (i.e. tube A located directly above the gantry 10 in Figs. 1A and 1B) to perform exposure, so as to obtain a front positioning scan image, a lateral positioning scan image and dual positioning scan images of the examination subject 20, specifically as follows:
As shown in Fig. 1A, when only a front positioning scan image is being acquired, tube A is located directly above the gantry to perform exposure, a detector a receives rays of tube A to obtain front projection data, and a front positioning scan image is subsequently obtained through image reconstruction.

As shown in Fig. 1B, when only a lateral positioning scan image is being acquired, tube A moves to a lateral position on the gantry to perform exposure, detector a receives rays of tube A to obtain lateral projection data, and a lateral positioning scan image is then obtained through image reconstruction.

When dual positioning scan images are being acquired, tube A is first located directly above the gantry to perform exposure, and detector a receives rays of tube A to obtain front projection data, then tube A moves to the lateral position on the gantry to perform exposure, and detector a receives rays of tube A to obtain lateral projection data, and then a front positioning scan image and a lateral positioning scan image (i.e. dual positioning scan images) are obtained through image reconstruction.

As can be seen, if only tube A is used for the positioning scan, time is needed for tube A to move to the target position in the course of performing the lateral positioning scan and especially the dual positioning scan, resulting in a long scan waiting time and low working efficiency, which are unsuitable for high-throughput clinical scenarios (with a large number of examination subjects).

In view of the above, an embodiment of the present invention provides a positioning scan method, applied to a dual-source CT apparatus. It should be explained that in this embodiment, the first tube and the second tube in the dual-source CT apparatus have automatically returned to initial positions before the positioning scan is performed; the initial position of the first tube is located directly above/below the gantry, and the initial position of the second tube is located at a left/right lateral position on the gantry, wherein "directly above/below" means the 12 o'clock/6 o'clock direction in the gantry, and "left/right lateral position" means the 3 o'clock/9 o'clock direction in the gantry. The statement "the first tube and the second tube have automatically returned to initial positions before the positioning scan is performed" means that the gantry rotates so that the first tube and the second tube return to their initial positions before the end of the previous scanning sequence or the beginning of the current scan. In an embodiment, the initial positions of the two tubes are as shown in Fig. 1A: the first tube is tube A located directly above the gantry 10, and the rays which it emits during exposure are received by detector a; the second tube is tube B located at the left lateral position on the gantry 10, and the rays which it emits during exposure are received by detector b.

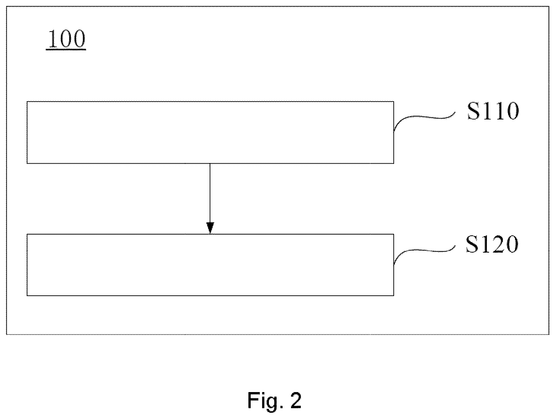

Fig. 2 shows a flow chart of a positioning scan method 100 in this embodiment. As shown in Fig. 2, the positioning scan method 100 comprises:
Step S110, receiving a positioning scan instruction; the type of the positioning scan instruction may be a front image scan instruction, or a lateral image scan instruction, or a dual positioning image scan instruction.
Step S120, performing the following operations according to the type of the positioning scan instruction:
   if it is a front image scan instruction, controlling the first tube to perform exposure, to obtain first front projection data;
   if it is a lateral image scan instruction, controlling the second tube to perform exposure, to obtain first lateral projection data;
   if it is a dual positioning image scan instruction, controlling the first tube and the second tube to simultaneously perform exposure, to obtain second front projection data and second lateral projection data respectively.

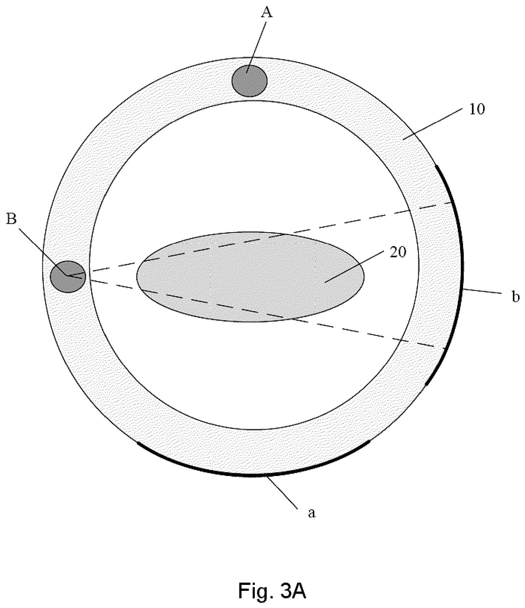

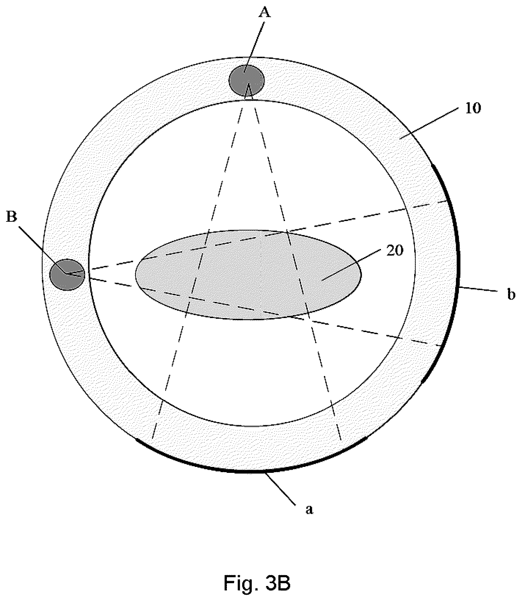

In this embodiment, when the front image scan instruction is received, exposure is performed by the first tube located directly above/below the gantry (referring to Fig. 1A, exposure is performed by tube A located directly above the gantry 10); when the lateral image scan instruction is received, exposure is performed by the second tube located at the left/right lateral position on the gantry (referring to Fig. 3A, exposure is performed by tube B located at the left lateral position on the gantry 10); when the dual positioning image scan instruction is received, exposure is performed simultaneously by the first tube located directly above/below the gantry and the second tube located at the left/right lateral position on the gantry (referring to Fig. 3B, tube A located directly above the gantry 10 and tube B located at the left lateral position on the gantry 10 perform exposure). Compared with the prior art, when performing a lateral image scan and a dual positioning image scan, there is no need for the first tube to move to a corresponding position, so the waiting time of the scan as a whole is reduced, thus increasing the overall positioning scan efficiency. Especially in the case of a dual positioning image scan, it is possible to obtain front projection data and lateral projection data simultaneously in a single exposure, significantly reducing the duration of scanning, and thus increasing the competitiveness of the dual-source CT apparatus in high-throughput clinical scenarios.

In this embodiment, the positioning scan method further comprises:
obtaining a first front positioning scan image by reconstruction according to the first front projection data, wherein a start position, a scan length and a dosage scheme of a subsequent Tomo scan can thus be determined according to the first front positioning scan image.

Moreover, a first lateral positioning scan image is obtained by reconstruction according to the first lateral projection data, wherein a start position, a scan length and a dosage scheme of a subsequent Tomo scan can thus be determined according to the first lateral positioning scan image.

In this embodiment, the positioning scan method further comprises: subjecting the second front projection data and the second lateral projection data to interference correction, to obtain the second front projection data and the second lateral projection data in corrected form; obtaining a second front positioning scan image by reconstruction on the basis of the second front projection data in corrected form, and obtaining a second lateral positioning scan image by reconstruction on the basis of the second lateral projection data in corrected form. A start position, a scan length and a dosage scheme of a subsequent Tomo scan can thus be determined according to the second front positioning scan image and the second lateral positioning scan image.

It will be understood that depth positions of the first tube and the second tube in the gantry are different; "depth position" means the distance between the tube and the examination subject, i.e. the relative positions of the tube and the center of the gantry (ISO-center). As shown in Fig. 1A, tube B (i.e. the second tube) is closer to the center of the gantry 10. Moreover, there is overlapping of rays when the first tube and the second tube perform exposure simultaneously, so the second front projection data and the second lateral projection data need to be subjected to interference correction. For details of the method of interference correction, an existing algorithm may be referred to; a superfluous description is not given here.

In addition, this embodiment further comprises: subjecting the second lateral positioning scan image to FOV (Field Of View) expansion correction, to enable it to be matched with the second front positioning scan image. By expanding the FOV of the second lateral positioning scan image, it is possible to avoid incomplete FOV coverage in the second lateral positioning scan image, so that subsequent determination of the scanning range will not be affected. At present, examples of FOV expansion correction methods include two different types of algorithms, namely linear expansion and AI-based expansion, which are not described here superfluously.

In this embodiment, when performing a dual positioning image scan, a first dose is used during exposure by the first tube, a second dose is used during exposure by the second tube, and the second dose is less than the first dose. It will be understood that the second tube is closer to the examination subject, and its rays are attenuated less, so the exposure dose of the second tube can be less than the exposure dose of the first tube, to reduce harm to the examination subject.

According to another aspect of the present invention, a positioning scan apparatus applied to a dual-source CT apparatus is provided, wherein a first tube and a second tube in the dual-source CT apparatus have automatically returned to their initial positions before a positioning scan is performed, and wherein the first tube is located directly above/below a gantry, and the second tube is located at a left/right lateral position on the gantry.

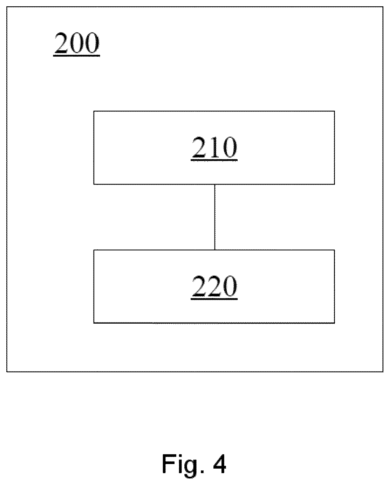

Fig. 4 shows a structural block diagram of a positioning scan apparatus 200 in this embodiment. As shown in Fig. 4, the positioning scan apparatus 200 comprises:
an instruction receiving module 210, configured to receive a positioning scan instruction;
an instruction executing module 220, configured to perform the following operations according to a type of the positioning scan instruction:
   if it is a front image scan instruction, controlling the first tube to perform exposure, to obtain first front projection data;
   if it is a lateral image scan instruction, controlling the second tube to perform exposure, to obtain first lateral projection data;
   if it is a dual positioning image scan instruction, controlling the first tube and the second tube to simultaneously perform exposure, to obtain second front projection data and second lateral projection data respectively.

It will be understood that the modules of the positioning scan apparatus 200 shown in Fig. 4 may correspond to the steps in the positioning scan method 100 described with reference to Fig. 2. Thus, the operations, features and advantages described above for the positioning scan method likewise apply to the positioning scan apparatus and the modules comprised therein. For conciseness, some operations, features and advantages are not described again here.

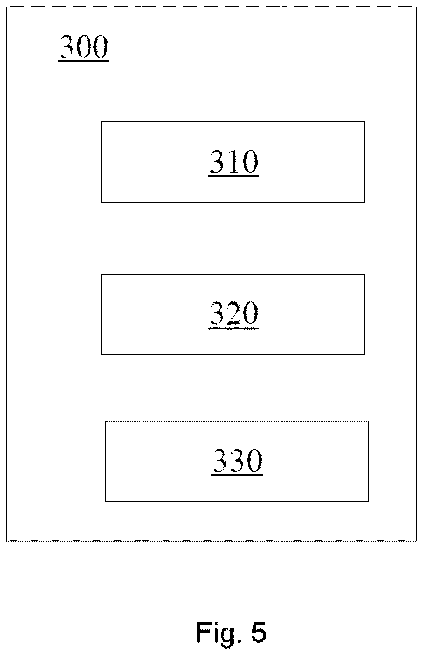

According to another aspect of the present invention, a dual-source CT apparatus is provided. Fig. 5 shows a structural block diagram of a dual-source CT apparatus 300 in this embodiment. As shown in Fig. 5, the dual-source CT apparatus 300 comprises: a first tube 310 and a second tube 320, which have automatically returned to initial positions before a positioning scan is performed, wherein an initial position of the first tube 310 is located directly above/below a gantry, and an initial position of the second tube 320 is located at a left/right lateral position on the gantry; and a controller 330, the controller 330 being configured to perform the method described in the present invention.

The dual-source CT apparatus 300 may further comprise other components, for example, a collimator which blocks some of the rays emitted by the first tube 310 and the second tube 320 for the purpose of imaging a desired imaging object region, and/or a ray detector which receives rays that have passed through the imaging object for the purpose of imaging the imaging object.

According to another aspect of the present invention, a non-transitory computer-readable storage medium storing a computer program is provided, wherein the computer program, when executed by a processor, realizes the method described in the present invention.

According to another aspect of the present disclosure, a computer program product is provided, comprising a computer program, wherein the computer program, when executed by a processor, realizes the method described in the present invention.

The above are merely preferred embodiments of the present invention, which are not intended to limit it. Any amendments, equivalent substitutions or improvements etc. made within the spirit and principles of the present invention shall be included in the scope of protection

## Claims

1. A positioning scan method, **characterized in that** it is applied to a dual-source CT apparatus, wherein a first tube and a second tube in the dual-source CT apparatus have automatically returned to initial positions before a positioning scan is performed, and wherein an initial position of the first tube is located directly above/below a gantry, and an initial position of the second tube is located at a left/right lateral position on the gantry;
the positioning scan method comprises:
receiving a positioning scan instruction;
performing the following operations according to a type of the positioning scan instruction:
if it is a front image scan instruction, controlling the first tube to perform exposure, to obtain first front projection data;
if it is a lateral image scan instruction, controlling the second tube to perform exposure, to obtain first lateral projection data;
if it is a dual positioning image scan instruction, controlling the first tube and the second tube to simultaneously perform exposure, to obtain second front projection data and second lateral projection data respectively.

2. The positioning scan method as claimed in claim 1, **characterized by** further comprising:
subjecting the second front projection data and the second lateral projection data to interference correction, to obtain the second front projection data and the second lateral projection data in corrected form;
obtaining a second front positioning scan image by reconstruction on the basis of the second front projection data in corrected form, and obtaining a second lateral positioning scan image by reconstruction on the basis of the second lateral projection data in corrected form.

3. The positioning scan method as claimed in claim 2, **characterized by** further comprising:
subjecting the second lateral positioning scan image to field-of-view expansion correction, to enable it to be matched with the second front positioning scan image.

4. The positioning scan method as claimed in claim 1, **characterized by** further comprising:
obtaining a first front positioning scan image by reconstruction according to the first front projection data.

5. The positioning scan method as claimed in claim 1, **characterized by** further comprising:
obtaining a first lateral positioning scan image by reconstruction according to the first lateral projection data.

6. The positioning scan method as claimed in claim 1, **characterized in that** when performing a dual positioning image scan, a first dose is used during exposure by the first tube, a second dose is used during exposure by the second tube, and the second dose is less than the first dose.

7. A positioning scan apparatus, **characterized in that** it is applied to a dual-source CT apparatus, wherein a first tube and a second tube in the dual-source CT apparatus have automatically returned to initial positions before a positioning scan is performed, and wherein the first tube is located directly above/below a gantry, and the second tube is located at a left/right lateral position on the gantry;
the positioning scan apparatus (200) comprises:
an instruction receiving module (210), configured to receive a positioning scan instruction;
an instruction executing module (220), configured to perform the following operations according to a type of the positioning scan instruction:
if it is a front image scan instruction, controlling the first tube to perform exposure, to obtain first front projection data;
if it is a lateral image scan instruction, controlling the second tube to perform exposure, to obtain first lateral projection data;
if it is a dual positioning image scan instruction, controlling the first tube and the second tube to simultaneously perform exposure, to obtain second front projection data and second lateral projection data respectively.

8. A dual-source CT apparatus, **characterized in that** the dual-source CT apparatus (300) comprises:
a first tube (310) and a second tube (320), which have automatically returned to initial positions before a positioning scan is performed, wherein an initial position of the first tube (310) is located directly above/below a gantry, and an initial position of the second tube (320) is located at a left/right lateral position on the gantry;
a controller (330), the controller (330) being configured to perform the method as claimed in any one of claims 1 - 6.

9. A non-transitory computer-readable storage medium storing a computer program, wherein the computer program, when executed by a processor, realizes the method as claimed in any one of claims 1 - 6.

10. A computer program product, comprising a computer program, wherein the computer program, when executed by a processor, realizes the method as claimed in any one of claims 1 - 6.
